# EUROPEAN PATENT APPLICATION

(11) **EP 3 692 831 A1**
(43) Date of publication of application: **12.08.2020**
(21) Application number: 19155861.8
(22) Date of filing: 07.02.2019
(51) Int. Cl.: A24F 47/00, A61M 15/06, A61M 11/04

(54) **SMOKING SUBSTITUTE DEVICE**

(71) Applicant: Nerudia Ltd., Liverpool, Merseyside L24 9HP (GB)
(72) Inventor: The designation of the inventor has not yet been filed
(74) Representative: Mewburn Ellis LLP

(57) **Abstract**

A smoking substitute system (101) having a fluid inlet (107), a fluid outlet (108), and an aerosol generator arranged between the inlet (107) and the outlet (108) and in fluid communication with each. The aerosol generator is operable to generate an aerosol from an aerosol precursor (104). The smoking substitute system (101) further comprises a heater (130) arranged between the inlet (107) and the aerosol generator, the heater being configured to heat fluid drawn through the inlet (107) towards the aerosol generator. A source of flavourant (132) is provided between the heater (130) and the aerosol generator so as to be presented to a flow of fluid from said heater (130) towards said aerosol generator, the flavourant comprising one or more substances that activate at least one of an olfactory receptor in a human nasal cavity and a taste receptor in a human oral cavity. A flow of fluid heated by the heater (130) is effective to release flavourant (132) from said source for entrainment in said fluid upstream of said aerosol generator.

## Description

### Field of the Invention

The present invention relates to a smoking substitute device and, in particular, a smoking substitute device that is able to deliver flavour to a user.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as an aerosol generator heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, e-liquid is heated by a heating device to produce an aerosol vapour which is inhaled by a user. Many e-cigarettes also deliver flavour to the user to enhance the experience. In such e-cigarettes, flavour compounds are contained in the e-liquid that is heated. However, toxicology restrictions are placed on the amount of flavour that can be contained in the e-liquid, and this can result in some e-liquid flavours delivering a weak and underwhelming taste sensation to consumers in the pursuit of safety. Further, there is a view that providing a flavourant as part of the e-liquid, such that the flavourant is vaporised with the e-liquid, may be disadvantageous.

There may be a need for improved design of smoking substitute systems, in particular in regards to the delivery of flavour to a user.

The present disclosure has been devised in the light of the above considerations.

### Summary of the Invention

At its most general, the present invention relates to provision of a heater to heat fluid passing through a smoking substitute device and thereby cause a flavourant to be entrained in the fluid flow.

According to a first aspect there is provided a smoking substitute device having a fluid inlet, a fluid outlet, and an aerosol generator arranged between the inlet and the outlet and in fluid communication with each, the aerosol generator operable to generate an aerosol from an aerosol precursor, wherein the smoking substitute device further comprises a heater arranged between the inlet and the aerosol generator, the heater being configured to heat fluid drawn through the inlet towards the aerosol generator, and a source of flavourant provided between the heater and the aerosol generator so as to be presented to a flow of fluid from said heater towards said aerosol generator, the flavourant comprising one or more substances that activate at least one of an olfactory receptor in a human nasal cavity and a taste receptor in a human oral cavity, and wherein a flow of fluid heated by the heater is effective to release flavourant from said source for entrainment in said fluid.

Optionally, the heater comprises one or more heating elements formed as a metal mesh.

Conveniently, the heater comprises one or more heater elements formed as coils.

Advantageously, the smoking substitute device is configured such that the aerosol generator and said heater are operable independently of one another.

Optionally, the aerosol generator is operable in combination with said heater.

Conveniently, the aerosol generator and said heater are operable in synchronism.

Advantageously, the smoking substitute device comprises a flavourant reservoir, wherein the flavourant is provided in the flavourant reservoir.

Optionally, the flavourant is provided as a liquid.

Conveniently, the source of flavourant is a flavoured article comprising a substrate carrying said flavourant.

Advantageously, at least part of said substrate is impregnated with the flavourant.

Optionally, at least part of the substrate is coated with the flavourant.

Conveniently, the source of flavourant is a flavour pod comprising a container at least partially filled with the flavourant.

Advantageously, the source of flavourant is a removable flavour part of the smoking substitute device.

Optionally, the removable flavour part is a replaceable part of the smoking substitute device.

Conveniently, the removable flavour part is a consumable.

Advantageously, a part of the smoking substitute device is a reusable part comprising a power source for the heater and aerosol generator.

Optionally, the heater is provided within the reusable part.

Conveniently, the source of flavourant is provided within the reusable part.

Advantageously, a part of the smoking substitute device is a removable precursor part comprising a source of aerosol precursor for the aerosol generator.

Optionally, the removable precursor part is a replaceable part.

Conveniently, the removable precursor part is a consumable.

Advantageously, the source of flavourant is provided in the removable precursor part.

Optionally, the source of flavourant is attachable to said removable precursor part.

Conveniently, the aerosol precursor is substantially free of flavourant.

Advantageously, the aerosol precursor is a liquid.

Optionally, the aerosol precursor contains nicotine.

According to a second aspect, a consumable may be provided comprising a source of flavourant for use with the smoking substitute device according to the first aspect.

According to a third aspect, a pack comprising a plurality of consumables according to the second aspect may be provided.

The flavourant may include one or more volatile substances. The flavourant may be provided in solid or liquid form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The smoking substitute apparatus may be in the form of a consumable. The consumable may be configured for engagement with a main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol precursor (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol precursor (e.g. e-liquid) of the system may be replenished by re-filling e.g. a precursor reservoir of the smoking substitute apparatus with the aerosol precursor (rather than replacing a consumable component of the apparatus).

In light of this, it should be appreciated that some of the features described herein as being part of the smoking substitute apparatus may alternatively form part of a main body for engagement with the smoking substitute apparatus (i.e. when the smoking substitute apparatus is in the form of a consumable).

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a precursor reservoir configured to store an aerosol precursor, such as an e-liquid. The e-liquid may, for example, comprise a base liquid and e.g. nicotine. The base liquid may include propylene glycol and/or vegetable glycerine. The e-liquid may be flavourless. That is, the e-liquid may not contain any flavourants and may consist solely of a base liquid of propylene glycol and/or vegetable glycerine and nicotine.

The precursor reservoir may be in the form of a tank. At least a portion of the tank may be translucent. For example, the tank may comprise a window to allow a user to visually assess the quantity of e-liquid in the tank. A housing of the smoking substitute apparatus may comprise a corresponding aperture (or slot) or window that may be aligned with a translucent portion (e.g. window) of the tank. The precursor reservoir may be referred to as a "clearomizer" if it includes a window, or a "cartomizer" if it does not.

The smoking substitute apparatus may comprise a passage for fluid flow therethrough. The passage may extend through (at least a portion of) the smoking substitute apparatus, between openings that may define an inlet and an outlet of the passage. The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage. In this respect, the tank may surround the passage.

The smoking substitute apparatus may comprise an aerosol-generator. The aerosol generator may comprise a wick. The aerosol generator may further comprise an aerosol generating heater. The wick may comprise a porous material. A portion of the wick may be exposed to fluid flow in the passage. The wick may also comprise one or more portions in contact with liquid stored in the precursor reservoir. For example, opposing ends of the wick may protrude into the precursor reservoir and a central portion (between the ends) may extend across the passage so as to be exposed to fluid flow in the passage. Thus, fluid may be drawn (e.g. by capillary action) along the wick, from the precursor reservoir to the exposed portion of the wick.

The aerosol generating heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. This vapour may subsequently cool to form an aerosol in the passage.

The smoking substitute apparatus (or main body engaged with the smoking substitute apparatus) may comprise a power source. The power source may be electrically connected (or connectable) to an aerosol generator of the smoking substitute apparatus (e.g. when engaged with the main body) and the heater of the smoking substitute apparatus. The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface may be configured to transfer electrical power from the power source to an aerosol generator of the consumable and the heater of the smoking substitute apparatus.

The electrical interface may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The smoking substitute apparatus or main body may comprise a controller, which may include a microprocessor. The controller may be configured to control the supply of power from the power source to the aerosol generator of the smoking substitute apparatus (e.g. via the electrical contacts) and the heater of the smoking substitute apparatus. A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. Wi-Fi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the aerosol generator of the consumable and/or the heater of the smoking substitute apparatus in response to a puff detection by the sensor. The control may be in the form of activation of the aerosol generator and/or the heater of the smoking substitute apparatus in response to a detected puff. That is, the smoking substitute apparatus may be configured to be activated when a puff is detected by the puff sensor. When the smoking substitute apparatus is in the form of a consumable, the puff sensor may form part of the consumable or the main body.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1 is a front view of a smoking substitute device and a main body in an engaged position;
Figure 2 is a front view of a smoking substitute device and a main body in a disengaged position;
Figure 3 is a section view of a smoking substitute device;
Figure 4 is a section view of a main body of a smoking substitute system comprising a heater;
Figure 5 is a section view of an exemplary flavourant reservoir having a wick extending into the fluid passage of a smoking substitute device;
Figure 6 is a section view of an exemplary flavourant reservoir having a fluid-permeable surface forming a peripheral wall of the fluid passage of a smoking substitute device;
Figure 7 is an end-on view of an exemplary flavourant reservoir having a plurality of bores extending therethrough;
Figure 8 is an enlarged section view of a section of a flavoured article;
Figure 9 is a section view of an exemplary flavour pod having a porous wick;
Figure 10 is a section view of an exemplary flavour pod having a fluid-permeable surface;
Figure 11 is an end-on view of an exemplary flavour pod having a plurality of bores extending therethrough;
Figure 12 is a front view of a smoking substitute device, main body, and flavour part in an engaged position;
Figure 13 is a front view of a smoking substitute device, main body and flavour part in a disengaged position; and
Figure 14 is a section view of a smoking substitute device comprising a heater and a flavourant reservoir.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

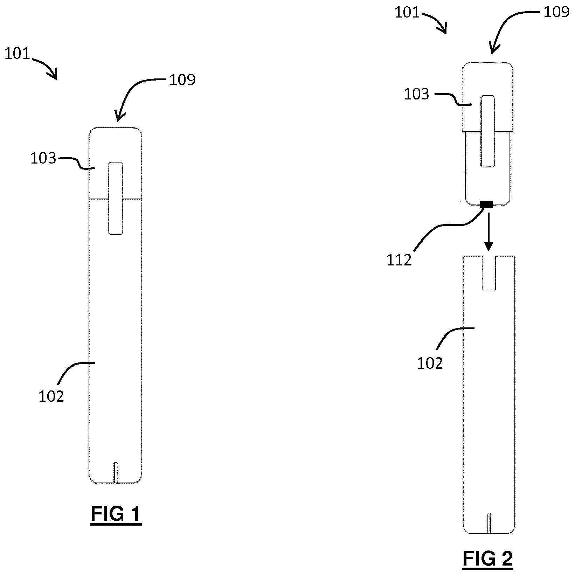

Figures 1 and 2 illustrate a smoking substitute system in the form of an e-cigarette system 101. The system 101 comprises an e-cigarette device defining a reusable main body 102 of the system 101, and a smoking substitute device in the form of an e-cigarette consumable (or "pod") 103, which may also be referred to as a precursor part. In the illustrated embodiment the consumable 103 (smoking substitute device) is removable from the main body (e-cigarette device), so as to be a replaceable component of the system 101. In other words, the e-cigarette system 101 is a closed system.

As is apparent from Figures 1 and 2, the consumable 103 is configured to engage the main body 102. Figure 1 shows the main body 102 and the consumable 103 in an engaged state, whilst Figure 2 shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol-former or aerosol precursor, which, in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless (and does not include any added flavourant). That is, if the e-liquid 104 were to be inhaled (i.e. in aerosol form) by a user, it would not have a particularly perceptible flavour or taste. It is to be appreciated, however, that in other embodiments the e-liquid 104 may comprise an inherent flavourant. In the present specification, the term flavourant may be understood as referring to one or more substances effective to activate at least one of an olfactory receptor in a human nasal cavity; and a taste receptor in a human oral cavity.

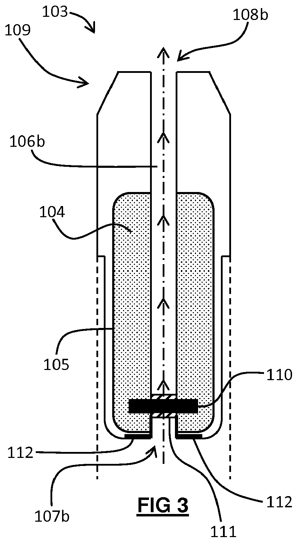

As is more apparent from Figure 3, the e-liquid 104 is stored within a precursor reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol precursor) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a fluid flow passage 106b that extends between an inlet 107b and an outlet 108 at opposing ends of the consumable 103. In this respect, the passage 106b comprises an upstream end at the end of the consumable 103 that engages with the main body 102, and a downstream end at an opposing end of the consumable 103 that comprises a mouthpiece 109 of the system 101. The fluid flow passage 106b comprises an inlet 107b at its upstream end, and an outlet at its downstream end, the outlet 108b thus forming part of, or being located at, the mouthpiece 109. When the consumable 103 is engaged with the main body 102, a user can inhale (i.e. take a puff) via the mouthpiece 109 so as to draw air through the passage 106b, and so as to form an airflow (indicated by arrows) in a direction from the inlet 107b to the outlet 108b of the passage 106b.

The passage 106b may be partially defined by a tube (e.g. a metal tube or a tube formed from plastic material) extending through the consumable 103. At its upstream inlet end, the passage 106b may be in fluid communication with a gap defined between the consumable 103 and the main body 102 when the consumable 103 and the main body 102 are interengaged, such that when a user draws on the outlet 108b at the mouthpiece 109, ambient air from outside of the system 101 is drawn into the passage 106b via the inlet 107b.

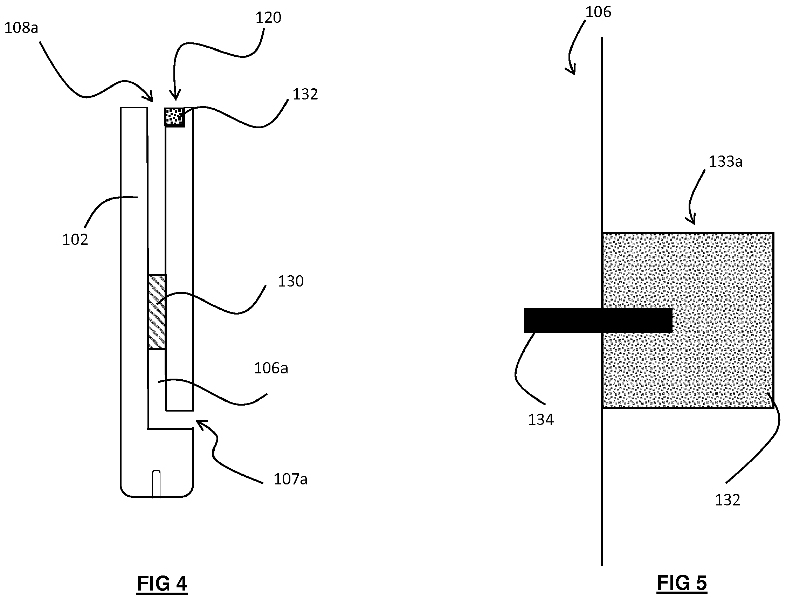

Alternatively, the main body 102 may additionally comprise a fluid passage 106a, extending from a main body inlet 107a to a main body outlet 108a as illustrated, for example, in Figure 4. In such an arrangement, the main body outlet 108a may be arranged such that when the main body 102 and consumable 103 are interengaged, a substantially fluid-tight seal is formed at the connection point between main body outlet 108a and fluid inlet 107b, meaning that main body fluid passage 106a and consumable passage 106b become interconnected to form a single passage 106 extending through the smoking substitute system 101 and fluidly connecting main body inlet 107a and consumable outlet 108b. Furthermore, the main body fluid passage 106a or the consumable fluid passage 106b may be shaped to provide a recess or receptacle 120 into which a source of flavourant 132 may be received, as will be described in more detail hereinafter.

The smoking substitute system 101 is configured to vaporise the e-liquid 104 for inhalation by a user. To provide this function, the consumable 103 is provided with an aerosol generator comprising a porous wick 110 and a resistive heating element in the form of a precursor heating filament 111 that is helically wound around a portion of the porous wick 110. The aerosol generator is arranged between the inlet 107a and the outlet 108b of the smoking substitute system 101, and is in fluid communication with both the inlet 107a and the outlet 108b. The porous wick 110 extends across the passage 106 (e.g. transverse to a longitudinal axis of the passage 106), and opposing ends of the wick 110 extend into the tank 105 so as to be submerged in the e-liquid 104 so as to draw e-liquid from the tank 105 via wicking or capillary action. In this way, e-liquid 104 contained in the tank 105 is conveyed from the opposing ends of the porous wick 110 to a central portion of the porous wick 110 so as to be exposed to the airflow in the passage 106 caused by a user inhaling or drawing on the mouthpiece 109. The helical filament 111 is wound about the exposed central portion of the porous wick 110 and is electrically connected to an electrical interface in the form of electrical contacts 112 mounted at the end of the consumable that is proximate the main body 102 (when engaged).

When the consumable 103 is engaged with the main body 102, the electrical contacts 112 make contact with corresponding electrical contacts (not shown) of the main body 102. The main body electrical contacts are electrically connected to a power source (not shown) of the main body 102, such that the filament 111 is electrically connected to the power source when the consumable 103 and the main body 102 are interengaged. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow past the wick 110 and, between the vaporisation point at the filament 111 and the outlet 108b of the passage 106, condenses to form an aerosol. This aerosol is then inhaled, via the mouthpiece 109, by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface.

In the illustrated smoking substitute system 101, an additional heater 130 (i.e. separate from and in addition to the heater of the aerosol generator) is provided upstream of the aerosol generator along a fluid passage 106 connecting the fluid inlet 107a or 107b and the aerosol generator. Further, a source of flavourant 132 is provided along the fluid passage 106, between the additional heater 130 and the aerosol generator, such that the source of flavourant 132 is presented to a flow of fluid from the additional heater 130 towards the aerosol generator. The arrangement is thus configured such that said flow of fluid is heated by the additional heater 130 to release flavourant 132 from the source for entrainment in said fluid upstream of the aerosol generator.

The flavourant 132 may be formulated such that heated fluid is required to release the flavourant 132 from the source. In other words, a fluid flow (for example from inhalation) without operation of the additional heater 130 would cause substantially no flavourant 132 to be entrained therein. This would allow, for example, for a user to select whether or not to use the flavourant during a vaping session. Further, this type of configuration may allow for control of intensity of the flavour, for example by varying the temperature of the additional heater 130.

Using the additional heater 130 to heat a fluid flow, said fluid flow then being flowed past the source of flavourant to heat it indirectly, rather than heating the source of flavourant 132 directly, may allow for a greater range of flavourants to be used in smoking substitute system 101. For example, it may allow the use of thermally sensitive flavourants 132 which could be degraded by the direct application of heat from a heater. Further, heating the fluid rather than heating the flavourant 132 directly may prolong the life of the additional heater 130 by preventing it from becoming fouled with residual flavourant 132.

The additional heater 130 may be operable in combination (for example in synchronism or with a predetermined operating time offset) with the heating filament 111 of the aerosol generator. Additionally or alternatively, the additional heater 130 and the heating filament 111 of the aerosol generator may be operable independently of one another.

In embodiments in which the e-liquid 104 contains an inherent flavourant, it will be appreciated that the release of flavourant 132 from the source in the manner noted above will serve to supplement, and optionally blend with, the flavourant of the e-liquid in use. For example, the flavourant 132 of the source may have the same flavour as that in the e-liquid 104 in order to supplement the flavour provided within the e-liquid and thereby provide the user with an intensified flavour sensation. Alternatively, the flavourant 132 of the source may be complementary to that in the e-liquid 104 (i.e. the flavours/aromas of the two flavourants, when mixed, provide a pleasing sensory combination to a user). Providing the flavourant 132 separately to the aerosol precursor 104 affords the user an opportunity to change between different flavourants 132 without necessitating a change of e-liquid, for example during a vaping session.

The additional heater 130 may comprise one or more heating elements such as resistive heating elements in the form of a mesh or a coil, around or through which fluid may flow. Such heating elements may extend partially or substantially completely across the passage 106 to maximise heat transfer from the or each element to the fluid. Alternatively, or additionally, the or each heating element may form a part of the peripheral wall of passage 106.

Various arrangements within the smoking substitute device 101, and specific configurations of both the additional heater 130 and the source of flavourant 132 are possible, some examples of which are described below.

For example, the additional heater 130 may be located in the main body 102, as illustrated in Figure 4. The configuration of the passage 106a illustrated in Figure 4 is for illustrative purposes only, and is not intended to be limiting. For example, the fluid inlet 107a might alternatively be located closer to or further away from the end of main body 102 which is configured to engage with the smoking substitute device 103 than illustrated, or may even be located on a different face of the main body 102. The recess 120 maybe omitted, or may be located at a different position along the fluid passage 106a. Other components of the main body 102 (e.g. the power source) are not illustrated in Figure 4 for clarity, but may be arranged to be adjacent to or surround all or part of the fluid passage 106a as required.

In the case of embodiments in which the additional heater 130 is provided within the main body 102 of the system, the source of flavourant 132 may take the form of a flavourant reservoir 133 located either in the main body 102, or as part of the consumable 103. The flavourant reservoir 133 may surround, and thus define, at least part of the passage 106. Alternatively, the flavourant reservoir 133 may be located adjacent to, but not surround the passage 106. The flavourant provided in such a flavourant reservoir 133 may be provided, for example, as a flavoured liquid. A reservoir 133 that is provided within the main body 102 may be user-refillable, and may thus be located proximate to the interface between main body 102 and the consumable pod 103 to allow convenient user-access for refilling. The flavourant reservoir may be at least partially filled during manufacture of the consumable103 such that the user is not required to fill the reservoir with flavourant before using the consumable.

The flavourant reservoir may further comprise one or more porous wicks 134 extending across at least part of fluid flow passage 106, so as to be presented to a fluid flow therethrough (e.g. transverse to a longitudinal axis of the passage 106), as illustrated exemplarily in Figure 5, where the reservoir is denoted as 133a. In embodiments where the flavourant reservoir 133a surrounds a portion of the passage 106, the porous wick 134 may extend substantially completely across the passage 106, such that both opposing ends of the wick extend into the flavourant reservoir 133a. Alternatively, in embodiments where the flavourant reservoir 133a does not surround the passage 106, only one end of each porous wick 134 may extend into the flavourant reservoir 133a, with the other end being located within the passage 106. The end or ends of the porous wicks 134 that extend into the flavourant reservoir 133a are arranged to be submerged in the flavourant 132. In this way, flavourant 132 contained in the flavourant reservoir 133a is conveyed from the end or ends of the porous wick 134 that extend into the flavourant reservoir 133a to a portion of the porous wick 134 located within the passage 106 so as to be exposed to the airflow therein (i.e. caused by a user inhaling).

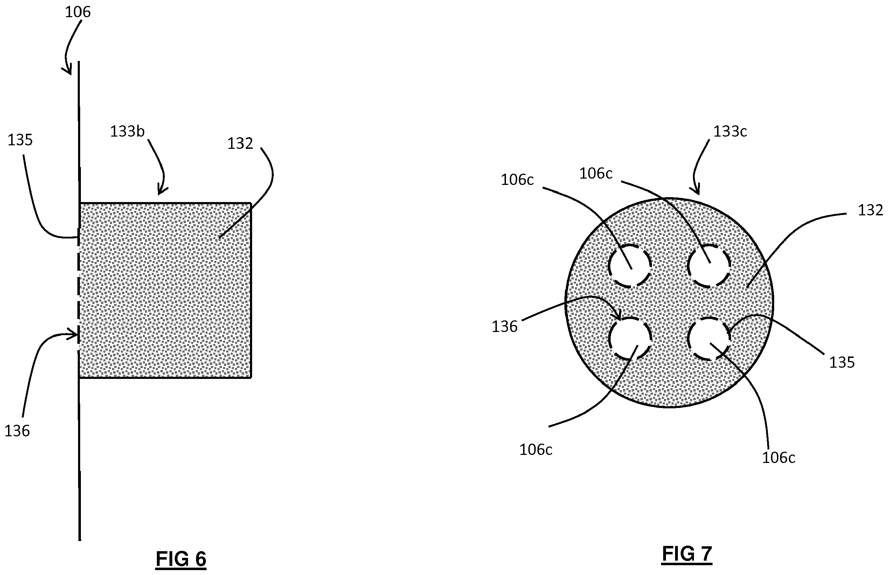

Alternatively or additionally, at least a part of a barrier separating the passage 106 from the flavourant reservoir 133b may be permeable to flavourant 132 as illustrated exemplarily in Figure 6. Figure 6 illustrates a perforated barrier 135 separating the passage 106 from the flavourant reservoir 133b. The barrier may thus comprise one or more small apertures 136 to permit the passage of flavourant therethrough, and may be formed as part of the sidewall of the flow passage 106. In this type of arrangement, flavourant 132 may permeate through the barrier from the flavourant reservoir 133b to the passage 106, for example by capillary action, so as to be exposed to the airflow in the passage 106 (i.e. caused by a user inhaling).

In an alternative arrangement illustrated in Figure 7, the flavourant reservoir 133c is housed within the flow passage 106 so as to extend substantially completely across the flow passage 106. As will be observed, in this arrangement, the reservoir 133c is provided with a plurality of relatively small diameter bores 106c, each of which extends fully through the reservoir from its upstream end to its downstream end. Each bore 106c may be defined by a similar permeable barrier 135c (each of which may thus be perforated with small apertures 136c) in a similar manner to the barrier arrangement described above with reference to Figure 6. As will be appreciated, fluid flow through the flow passage 106 will thus become split into separate flow streams through the bores 106c of the reservoir, the flow streams then rejoining and mixing with one another downstream of the reservoir. This type of multi-bore configuration of the reservoir 133c can be effective to increase the effective surface area through which the flavourant can permeate, when compared to the single barrier arrangement of Figure 6, which can be effective to increase the concentration of flavourant which may be entrained in the airflow through the passage 106.

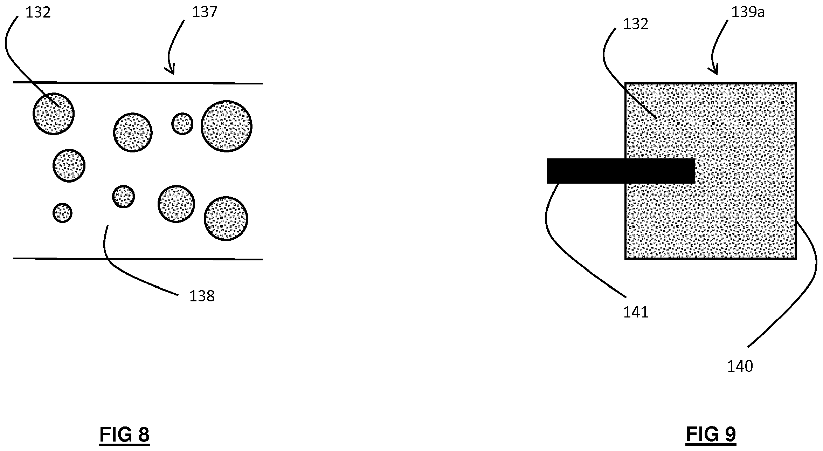

Alternatively, with reference to Figure 8, in embodiments where the additional heater 130 is provided within the main body 102 of the system, it is envisaged that the source of flavourant 132 may be a flavoured article 137, comprising a substrate 138 carrying the flavourant 132. Such a flavoured article 137 may be received within the fluid flow passage 106, which may be shaped to provide a recess or receptacle 120 within which the flavoured article 137 may be located. At least part of the substrate 138 may be formed from a polymeric material (e.g. silicone). Further, at least part of the substrate 138 may be formed from a porous material, foam or foamed material as illustrated exemplarily in Figure 8. Still further, at least a part of the substrate 138 may be formed from an air permeable material. The flavourant 132 may be one or both of comprised within or coated on a surface of the substrate 138. The flavourant 132 may be introduced into the flavoured article 137 by coating (e.g. spray coating). Alternatively or additionally, the flavourant 132 may be introduced or impregnated into the flavoured article 137 by, for example, immersion of the substrate 138 in a liquid comprising flavourant 132. This process of introducing flavourant 132 could either be carried out during manufacture of the flavoured article 137, or by an end-user.

Alternatively, having regard to Figure 9, the source of flavourant 132 may be a "flavour pod" 139 comprising a container 140 at least partially filled with the flavourant 132. Similarly to the flavoured article 137 illustrated in Figure 8, the flavour pod 139 may be received within the passage 106, which may be shaped to provide a recess or receptacle 120 into which the flavour pod 139 may be located. When in place, a surface of the flavour pod 139b may define part of a peripheral wall of the passage 106. The flavour pod 139a may furthermore comprise one or more porous wicks 141 extending at least partially across the passage 106 (e.g. transverse to a longitudinal axis of the passage 106), so as to be presented to a fluid flow therethrough. At least one end of each porous wick 141 extends into the flavour pod container 140, so as to be submerged in the flavourant 132. In this way, the flavourant 132 contained in the flavour pod container 140 is conveyed from the end or ends of the porous wick 141 extending into the flavour pod container 140 to a portion of the porous wick 141 located within the passage 106, so as to be exposed to the airflow therein (i.e. caused by a user inhaling).

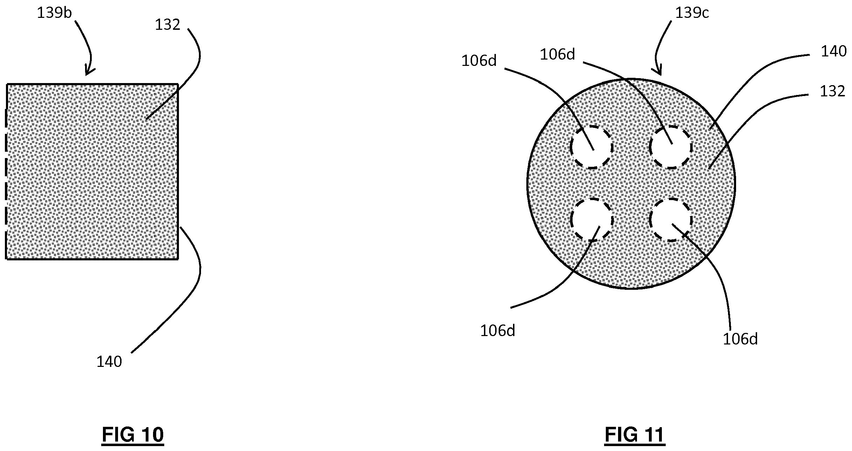

Additionally or alternatively, at least a part of a surface of the flavour pod 139 located adjacent to, or defining part of passage 106 may be flavourant-permeable as illustrated exemplarily in Figure 10. Alternatively, the flavour pod 139c may have a plurality of fluid passages or bores 106d therethrough, at least part of the wall or barrier dividing each bore 106d from the flavourant container 140 being flavourant-permeable as illustrated exemplarily in Figure 11. As will be appreciated by those of skill in the art, these arrangements of Figures 10 and 11 bear similarities with the arrangements of Figures 6 and 7. In such arrangements, flavourant 132 may permeate from the flavour pod 139 to the passage 106 for entrainment in the airflow therethrough (i.e. caused by a user inhaling).

The recess or receptacle 120 in which flavoured article 137 or flavour pod 139 is locatable may be provided within the main body 102, the system 101, or within the consumable 103. The flavoured article 137 or flavour pod 139 may be releasably attached to the consumable 103, for example to the end of the consumable which is configured to engage with the main body 102. Various means of attachment between the source of flavourant 132 and the consumable 103 may be possible, including, but not limited to, an interference fit, a snap fit comprising one or more raised bumps and corresponding recesses on the interfacial surface between the two components, or through physically coupling together by screwing one onto the other, through a bayonet fitting, through a latching mechanism, or through a clip or clasp. If the source of flavourant 130 is attached via a latching mechanism, this may further include a button by which the latch may be released. A clip or clasp may be fixed in position relative to the consumable 103, or hingedly, rotatably, or slideably attached to the consumable 103 so as to be movable to allow the flavoured article 137 or flavour pod 139 to be fitted and released.

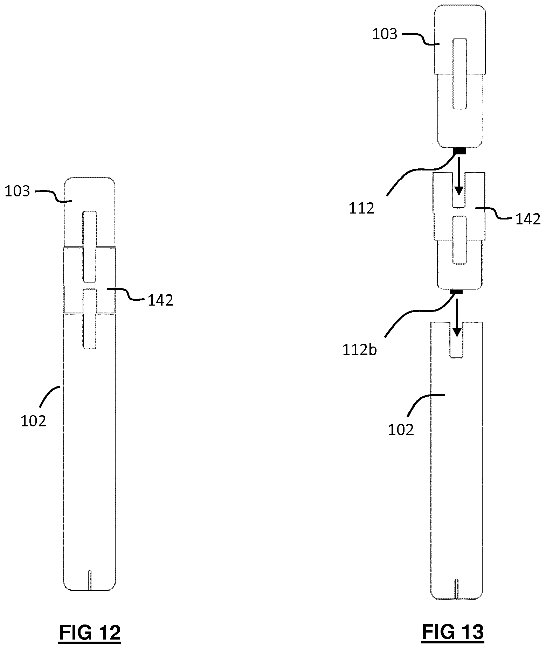

The flavoured article 137 or flavour pod 139 may alternatively be comprised within a flavour part 142 of the smoking substitute system 101, in the manner illustrated schematically in Figures 12 and 13. Said flavour part 142 may be located between the consumable 103 and the main body 102, such that each engages with an opposing end or side of the flavour part 142, but not directly with each other. The flavour part 142 in such an embodiment may further comprise an electrical contact 112b and connection therethrough, so that power may be conveyed from the contacts of the main body 102 to the contacts 112 of the consumable 103, via the flavour part 142. The interface between the consumable 103 and flavour part 142 may be configured to be the same as the interface between the flavour part 142 and main body 102, such that the user is afforded the option of choosing to use the smoking substitute device 101 either with or without the flavour part 142.

The flavoured article 137 or flavour pod 139 may therefore be provided as a stand-alone consumable, independent of the consumable 103 comprising the aerosol generator. One or more flavoured articles 137 or flavour pods 139 may be provided in a retail pack. Each flavoured article 137 or flavour pod 139 within a pack may comprise the same flavourant 132. Alternatively, the pack may comprise flavoured articles 137 or flavour pods 139 containing different flavourants 132, in order to provide a user with a selection of possible flavourants 132.

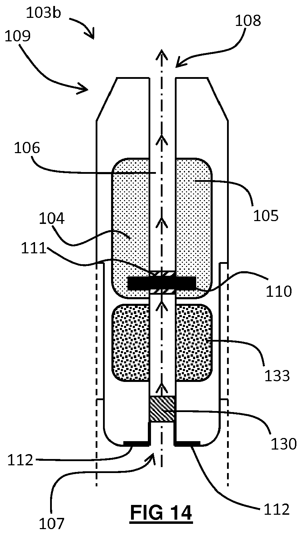

In alternative embodiments, it is envisaged that the additional heater 130 may be provided within a consumable 103b, located along the fluid passage 106b between fluid inlet 107b and the aerosol generator of the consumable, as illustrated exemplarily in Figure 14. In such an embodiment, the source of flavourant 132 could be a flavourant reservoir 133. In such a configuration, the main body 102 may not comprise a fluid inlet 107a, outlet 108a or passage 106a.

One or more consumables 103b may be provided in a retail pack. Each consumable 103b within a pack may comprise the same flavourant 132. Alternatively, the pack may comprise consumables 103b containing different flavourants 132, in order to provide a user with a selection of possible flavourants 132.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute system (101) having a fluid inlet (107), a fluid outlet (108), and an aerosol generator arranged between the inlet (107) and the outlet (108) and in fluid communication with each, the aerosol generator operable to generate an aerosol from an aerosol precursor (104); **characterized in that**:
the smoking substitute system (101) further comprises:
a heater (130) arranged between the inlet (107) and the aerosol generator, the heater being configured to heat fluid drawn through the inlet (107) towards the aerosol generator, and
a source of flavourant (132) provided between the heater (130) and the aerosol generator so as to be presented to a flow of fluid from said heater (130) towards said aerosol generator,
the flavourant comprising one or more substances that activate at least one of:
an olfactory receptor in a human nasal cavity; and
a taste receptor in a human oral cavity, and wherein
a flow of fluid heated by the heater (130) is effective to release flavourant (132) from said source for entrainment in said fluid upstream of said aerosol generator.

2. A smoking substitute system (101) according to claim 1, wherein
the heater (130) comprises one or more heating elements formed as a metal mesh.

3. A smoking substitute system (101) according to claim 1 or claim 2, wherein
the heater (130) comprises one or more heater elements formed as coils.

4. A smoking substitute system (101) according to any one of the preceding claims, wherein
the smoking substitute system (101) is configured such that the aerosol generator and said heater (130) are operable independently of one another.

5. A smoking substitute system (101) according to any one of the preceding claims, wherein
the aerosol generator is operable in combination with said heater (130).

6. A smoking substitute system (101) according to any one of the preceding claims, wherein
the smoking substitute system (101) comprises a flavourant reservoir (133); and wherein
the flavourant (132) is provided in the flavourant reservoir (133).

7. A smoking substitute system (101) according to any one of claims 1 to 5, wherein
said source of flavourant (132) is a flavoured article (137) comprising a substrate (138) carrying said flavourant (132).

8. A smoking substitute system (101) according to any one of claims 1 to 5, wherein
said source of flavourant (132) is a flavour pod (139) comprising a container (140) at least partially filled with the flavourant (132).

9. A smoking substitute system (101) according to claim 7 or claim 8, wherein
said source of flavourant (132) is a removable flavour part (142) of the smoking substitute system (101).

10. A smoking substitute system (101) according to claim 9, wherein
said removable flavour part (142) is a consumable.

11. A smoking substitute system (101) according to any one of the preceding claims, wherein
a part of the smoking substitute system (101) is a reusable part (102) comprising a power source for the heater (130) and aerosol generator.

12. A smoking substitute system (101) according to claim 11, wherein
said heater (130) is provided within said reusable part (102).

13. A smoking substitute system (101) according to any of the preceding claims, wherein
a part of the smoking substitute system (101) is a removable precursor part (103) comprising a source of aerosol precursor (104) for the aerosol generator.

14. A smoking substitute system (101) according to claim 13, wherein
said removable precursor part (103) is a consumable.

15. A smoking substitute system (101) according to any one of the preceding claims, wherein
the aerosol precursor (104) is substantially free of flavourant.
